Europäisches Patentamt

European Patent Office

Office européen des brevets

(11) Publication number: **0 242 128 B1**

(12) **EUROPEAN PATENT SPECIFICATION**

(45) Date of publication of patent specification: **06.11.91**  (51) Int. Cl.⁵: **A61B 5/20, G01F 1/52**

(21) Application number: **87303108.2**

(22) Date of filing: **09.04.87**

(54) **Urine meter.**

(30) Priority: **12.04.86 GB 8608974**

(43) Date of publication of application:
**21.10.87 Bulletin 87/43**

(45) Publication of the grant of the patent:
**06.11.91 Bulletin 91/45**

(84) Designated Contracting States:
**AT BE CH DE ES FR GB IT LI LU NL SE**

(56) References cited:
**EP-A- 0 008 450**
**EP-A- 0 118 066**
**US-A- 3 831 453**
**US-A- 4 305 405**

(73) Proprietor: **SHERWOOD MEDICAL COMPANY**
**1831 Olive Street**
**St. Louis, MO 63103(US)**

(72) Inventor: **Katsaros, Georges**
**4 R. Mathieu Bodson**
**B-4500 Jupille(BE)**
Inventor: **Macors, Paul**
**132 Rue Naniot**
**B-4000 Liege(BE)**

(74) Representative: **Porter, Graham Ronald et al**
**c/o Wyeth Laboratories Huntercombe Lane**
**South**
**Taplow Maidenhead Berkshire SL6 0PH(GB)**

## Description

This invention relates to urine meters

The rate of urine flow reflects both cardiac output and renal function. Urine flow measurement is therefore an important non-invasive technique in determining a patient's physiological condition, for example after major surgery or in the treatment of shock or burns. Known devices to collect urine have measurement chambers to allow monitoring staff to record the volume of urine passed in a predetermined time.

Where urine meters are employed, the patient's bladder is generally catheterised and the open end of the catheter connected to an inlet port of the meter. Prior art meters have employed several measuring chambers arranged to act sequentially. For instance EP-A-118066 discloses urine meters having a substantially upright primary chamber and one or more overflow chambers. In the known meters, a primary measurement chamber may have a small volume but be calibrated to measure low bladder output very accurately, the primary chamber may have a capacity of 30ml and be calibrated in 1ml steps. An overflow measurement chamber, which overflows from the primary chamber, may have a capacity of 200ml and be calibrated in 10ml steps. A second overflow chamber may constitute a collection bag and have a capacity of 2000ml calibrated in 100ml steps.

The primary and overflow chambers constitute the meter and may be emptied into the collection bag after each recording of volume is made. In this way accurate measurement of urine flow can be recorded by the medical staff.

Urine meters should have other desirable features. For example the meter should include a drip chamber at the inlet to prevent bacteria entering the bladder through the catheter and causing infection. The primary chamber should have an outlet from which a sample can be drawn for analysis. The collection bag should have a drain tap to allow the bag to be emptied.

One problem with prior art urine meters is that the dimensions of the primary measurement chamber must meet certain physical constraints and consequently the primary chamber may be smaller or less accurately calibrated than otherwise desirable. Ideally the primary chamber should be a long passage of small cross-sectional area; such a chamber would permit accurate volume measurement. The primary chamber must not however be so long and narrow that it is fragile or difficult and expensive to manufacture. Preferably the primary chamber is located within the more rigid urine meter body forming the fluidly connected primary and overflow chambers both for physical protection from knocks and to give desirable overflow characteristics. The urine meter and collection bag should not be unduly large or bulky since the assembly must be hung on the patient's bed at a sufficiently low level to allow gravity drainage.

One further problem with prior art urine meters is that if the primary chamber overflows into the overflow chamber by a small amount it is difficult to determine with accuracy the rate of flow since the overflow chamber is not calibrated to the same scale.

The present invention provides an improved urine meter having a large primary collection chamber susceptible of accurate calibration and which nevertheless meets the aforementioned parameters.

According to the invention there is provided a urine meter having a primary measurement chamber with an inlet at one open end and an overflow measurement chamber characterised in that the primary chamber has an outlet at the other open end in fluid communication with the overflow measurement chamber and the primary chamber extends around the periphery of the overflow chamber.

In this way the length of the primary chamber is determined by the size of the overflow chamber but is nevertheless at least twice as long as prior art primary chambers. The accuracy of the primary chamber is determined by its cross-sectional area which can be smaller than hitherto and therefore more accurate because of the increased length. Alternatively the primary chamber can have the same accuracy of measurement and a larger volume.

Preferably the primary chamber is generally 'V' shaped and has two upright limbs. The limbs are open at the upper ends thereof, one limb forming the inlet and the other the overflow into the overflow chamber. The limbs may be defined by a partition wall between two halves of a rigid plastic moulding which give a relatively straightforward and economical construction.

The primary chamber preferably has no horizontal portion and is provided with an outlet at the lowest point. This arrangement ensures complete draining of the primary chamber for sampling purposes.

The overflow chamber may be sub-divided by a weir to define two measurement sub-chambers which overflow sequentially from the primary chamber.

As previously mentioned the urine meter is preferably 'V' shaped. The overflow chamber thus narrows towards the bottom thereof and gives the possibility of greater accuracy of measurement, at least when the primary chamber initially overflows. Where the overflow chamber is sub-divided the accuracy of measurement is improved since the ratio of cross-sectional area to depth is further reduced.

The urine meter hangs in front of a flexible collection bag and is preferably connected thereto by a rigid tube which spaces the meter and bag by a distance sufficient to avoid tipping of the meter as the collection bag fills and consequently swells. The tube may be an elbow from which the collection bag hangs. Alternatively a bed hook with spaced hanging positions for the bag and meter can be provided. The bed hook for the meter may be provided with means for locating and supporting the meter inlet tube.

Other aspects of the invention will be apparent from the following description of a preferred embodiment shown by way of example only in the accompanying schematic illustration in which:-

Figure 1 is a side elevation of a urine meter assembly according to the invention;

Figure 2 is a transverse section through the urine meter of Figure 1 and showing the collection bag in dotted outline;

Figure 3 illustrates the method of emptying the urine meter into the collection bag; and

Figure 4 illustrates a urine meter assembly and alternative bed hook.

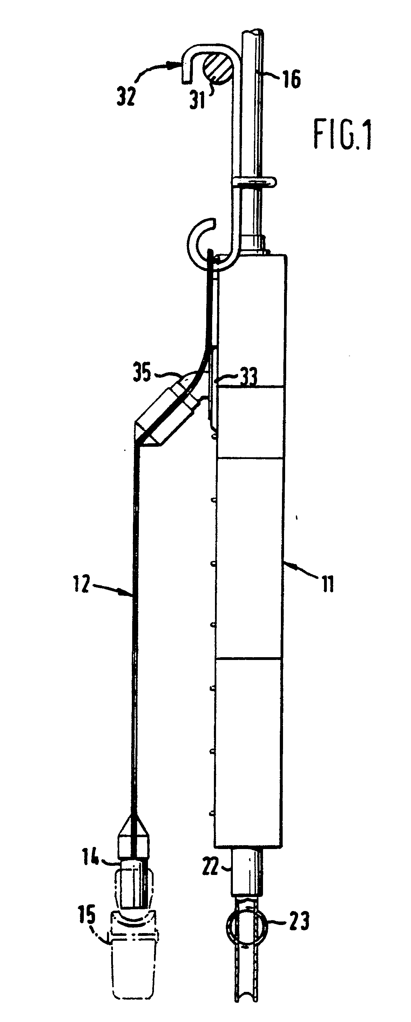

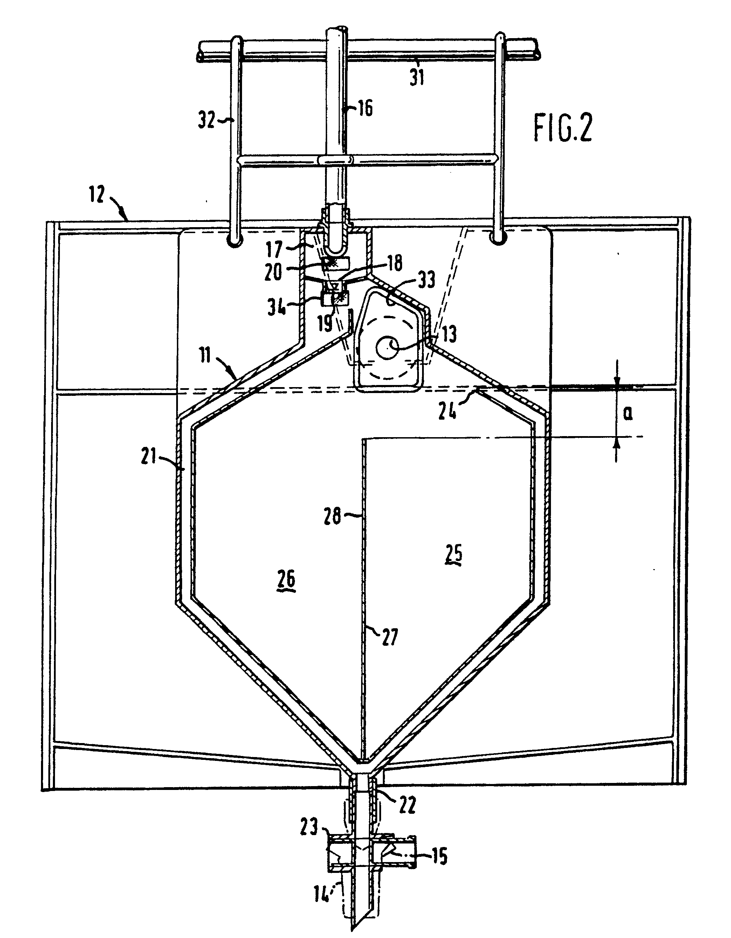

With reference to Figures 1 and 2 there is illustrated a urine meter 11 having a collection bag 12 and communicating aperture 13. The collection bag 12 has an outlet 14 normally closed by a drain tap 15. The urine meter has an inlet tube 16 which is connected, in use, to a catheter inserted into a patient for bladder drainage. The inlet tube opens into a drip chamber 17 having an orifice 18 through which urine flows. Exit from the drip chamber is through a non-return valve indicated by the schematic arrow. The drip chamber ensures that the flow of urine is discontinuous and that bacteria cannot therefore use the flow path as an entry to the bladder.

A hydrophobic filter 20 is provided in a side wall of the drip chamber to prevent excessive back pressure in the connecting tube 16 and hence in the patient's bladder. The filter may be of a known type which prevents entry of bacteria.

Urine flows from the drip chamber 17 through the non-return valve 19, into a three compartment collection chamber. Urine flows firstly into a primary chamber 21 which extends around the periphery of the urine meter body as shown. The chamber 21 constitutes the primary measurement chamber of the meter and has a sampling outlet 22 normally closed by the drain tap 23.

The right hand limb of the primary chamber, as viewed, is open and constitutes a weir 24 over which urine flows into an overflow sub-chamber 25 when the primary chamber is full.

The sub chamber 25 is separated from an associated sub-chamber 26 by a central wall 27 which constitutes another weir 28 over which fluid from the sub-chamber 25 can overflow. The weir 24 is higher than weir 28 as indicated on Figure 2 by the dimension 'a', to avoid backflow from chamber 25 into chamber 21 when the sampling valve 22 is opened. The arrangement of weirs ensures sequential filling of the sub-chambers.

The collection chamber is vented via a second hydrophobic filter 34 which ensures rapid emptying of the contents into the collection bag 12.

Once the sub-chamber 26 is filled with urine the level increases until urine flows through the aperture 13 into the collection bag 12. The aperture 13 is below the level of the drip chamber. The bag is connected to a rigid elbow 35 of the collection chamber; the elbow separates the chamber and bag sufficient to allow the bag to expand in use without tipping the chamber.

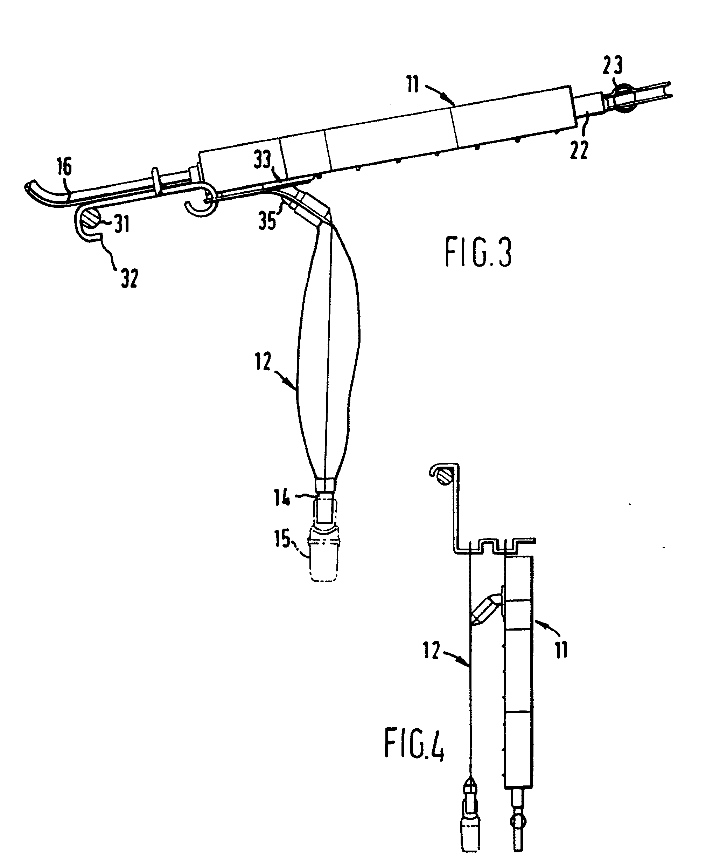

The urine meter assembly may be secured to a bed rail 31 by a suitable means, for example hook 32, as also illustrated in Figure 3. The hook 32 is shaped to locate the support inlet tube 16 as shown.

The urine meter is preferably manufactured from a relatively hard rigid thermoplastic material, for example, an acrylic, polystyrene or polycarbonate plastic whereas the collection bag is preferably of PVC or a similar relatively soft and flexible material. The urine meter should be sufficiently transparent to allow readings to be made through the walls thereof.

In use urine flows through the drip chamber 17 and non-return valve 19 into the primary measurement chamber 21. The chamber is relatively long compared with prior art measurement chambers and can thus be calibrated to give very accurate volume measurements. Clearly the accuracy will depend on the actual length of the chamber and the cross-sectional area but in a preferred embodiment the total volume of the chamber may be 50ml which gives both improved capacity and accuracy over prior art primary measurement chambers.

Graduations provided on the meter body allow the volume of urine in the primary chamber to be recorded. Since both limbs of the primary chamber fill equally the graduation need only be provided on one limb, preferably the right limb as viewed.

If the primary chamber fills to the level of weir 24 urine will overflow into the sub-chamber 25. Graduations are provided on the meter to allow volume of fluid in chamber 25 to be recorded. The base of chamber 25 is tapered and this allows the initial overflow of urine to be accurately measured since the cross-sectional area of the base of sub-chamber 25 is small.

If the sub-chamber 25 fills, urine overflows into sub-chamber 26 which also has a tapered base. If the sub-chamber 26 fills urine will overflow through the aperture into the collection bag 12.

Where accurate measurements of urine flow are required, an attendant will record the volume of urine in the primary chamber at regular intervals. Once a reading has been taken the meter can be emptied into the collection bag by tipping the meter as shown in Figure 3. Urine will flow through the aperture 13 until the meter is empty.

As best seen from Figure 1, the aperture 13 lies at the base of a recessed collection chamber 33 which ensures complete emptying of the urine meter into the collection bag.

The left hand limb of the primary chamber 21, as viewed, is open to ensure complete evacuation. The inner wall of this limb extends above the level of the aperture 13 to prevent accidental transfer of urine from the primary chamber 21 to the overflow chambers 25, 26 if, for example, the meter is knocked or is not hanging vertically. Care must be taken that urine meters are hung vertically and the hook arrangement and weight of the meter usually ensures this condition. Clearly no meter can be entirely accurate if not in an upright position since the graduations on the face of the meter rely on this condition. A further advantage of the present construction is that a non-vertical meter will be apparent by the different levels of urine in the two limbs of the primary chamber.

Figure 4 illustrates an alternative bed hook having spaced hook portions to support the meter and collection bag at a sufficient distance apart to allow the bag to swell.

Other constructions and arrangements of the invention are possible and it is intended that the scope of this specification be determined by reference to the appended claims.

## Claims

1. A urine meter having a primary measurement chamber (21) with an inlet at one open end and an overflow measurement chamber (25,26) characterised in that the primary chamber has an outlet at the other open end in fluid communication with the overflow measurement chamber (25, 26) and the primary chamber (21) extends around the periphery of the overflow chamber (25,26).

2. A urine meter according to Claim 1, wherein the primary chamber is substantially 'V' shaped and has two generally upright limbs.

3. A urine meter according to Claim 2 wherein one of said limbs is an inlet limb and the other is an overflow outlet limb.

4. A urine meter according to Claim 3, wherein said outlet limb is open at the upper end thereof for fluid communication with the overflow chamber.

5. A urine meter according to any preceding Claim, wherein the overflow chamber (25,26) is sub-divided by a weir (28).

6. A urine meter according to Claim 5, wherein the overflow level of said weir (28) is below the overflow level (24) of said primary chamber.

7. A urine meter according to any preceding Claim, wherein the primary chamber is provided with a drain tap (23).

8. A urine meter according to any preceding Claim, wherein the overflow chamber (25, 26) is tapered at the base thereof.

9. A urine meter according to any preceding claim and having an overflow outlet pipe (35) to an overflow bag (12), the pipe spacing said meter and bag sufficient to allow expansion of the bag in use.

## Revendications

1. Dispositif de mesure d'urine comportant une chambre de mesure principale (21) présentant une entrée à une extrémité ouverte et une chambre de mesure de trop-plein (25, 26), caractérisé en ce que la chambre principale comporte une sortie, à son autre extrémité ouverte, en communication de fluide avec la chambre de mesure de trop-plein (25, 26), et la chambre principale (21) s'étend autour de la périphérie de la chambre de trop-plein (25, 26).

2. Dispositif de mesure d'urine suivant la revendication 1, dans lequel la chambre principale est en substance en "V" et comporte deux branches généralement verticales.

3. Dispositif de mesure d'urine suivant la revendication 2, dans lequel une des branches est une branche d'entrée et l'autre est une branche de sortie par trop-plein.

4. Dispositif de mesure d'urine suivant la revendication 3, dans lequel la branche de sortie est ouverte à son extrémité supérieure en vue d'une communication de fluide avec la chambre de trop-plein.

5. Dispositif de mesure d'urine suivant l'une quelconque des revendications précédentes, dans lequel la chambre de trop-plein (25, 26) est subdivisée par un seuil de trop-plein (28).

6. Dispositif de mesure d'urine suivant la revendication 5, dans lequel le niveau de trop-plein du seuil (28) est situé en dessous du niveau de trop-plein (24) de la chambre principale.

7. Dispositif de mesure d'urine suivant l'une quelconque des revendications précédentes, dans lequel la chambre principale porte un robinet d'évacuation (23).

8. Dispositif de mesure d'urine suivant l'une quelconque des revendications précédentes, dans lequel la chambre de trop-plein (25, 26) est façonnée en pointe à sa base.

9. Dispositif de mesure d'urine suivant l'une quelconque des revendications précédentes, comportant un tube de sortie de trop-plein (35) raccordé à une poche de trop-plein (12), le tube espaçant le dispositif de mesure et la poche suffisamment pour permettre à la poche de se dilater en service.

**Patentansprüche**

1. Urinmeßgerät mit einer Hauptmeßkammer (21) mit einem Einlaß an einem offenen Ende, und mit einer Überlaufmeßkammer (25, 26), dadurch gekennzeichnet, daß die Hauptkammer einen Auslaß am anderen offenen Ende in Fluidverbindung mit der Überlaufmeßkammer (25, 26) hat und sich die Hauptkammer (21) rund um den Umfang der Überlaufkammer (25, 26) erstreckt.

2. Urinmeßgerät nach Anspruch 1, bei dem die Hauptkammer im wesentlichen V-förmig ist und zwei allgemein aufrechte Schenkel hat.

3. Urinmeßgerät nach Anspruch 2, wobei einer der Schenkel ein Einlaßschenkel und der andere ein Überlaufauslaßschenkel ist.

4. Urinmeßgerät nach Anspruch 3, bei dem der Auslaßschenkel an seinem oberen Ende für eine Fluidverbindung mit der Überlaufkammer offen ist.

5. Urinmeßgerät nach einem der vorhergehenden Ansprüche, bei dem die Überlaufkammer (25, 26) durch ein Wehr (28) unterteilt ist.

6. Urinmeßgerät nach Anspruch 5, bei dem der Überlaufpegel des Wehrs (28) unter dem Überlaufpegel (24) der Hauptkammer liegt.

7. Urinmeßgerät nach einem der vorhergehenden Ansprüche, bei dem die Hauptkammer mit einer Ablaufanzapfung (23) versehen ist.

8. Urinmeßgerät nach einem der vorhergehenden Ansprüche, bei dem die Überlaufkammer (25, 26) an ihrem Unterteil verjüngt ist.

9. Urinmeßgerät nach einem der vorhergehenden Ansprüche und mit einem Überlaufauslaßrohr (35) zu einem Überlaufbeutel (12), wobei das Rohr das Meßgerät und den Beutel ausreichend auf Abstand hält, um bei Gebrauch eine Ausdehnung des Beutels zu ermöglichen.

FIG.1

FIG.2

FIG.3

FIG.4